# EUROPEAN PATENT SPECIFICATION

(11) **EP 4 475 943 B1**
(45) Date of publication and mention of the grant of the patent: **23.09.2026**
(21) Application number: 23702861.8
(22) Date of filing: 07.02.2023
(51) Int. Cl.: A61N 1/375

(54) **ACTIVE IMPLANTABLE MEDICAL DEVICE COMPRISING A FILM-LIKE CONNECTING ELEMENT**
AKTIVE IMPLANTIERBARE MEDIZINISCHE VORRICHTUNG MIT EINEM FOLIENARTIGEN VERBINDUNGSELEMENT
DISPOSITIF MÉDICAL IMPLANTABLE ACTIF COMPRENANT UN ÉLÉMENT DE CONNEXION DE TYPE FILM

(30) Priority: 11.02.2022 EP 22156255
(43) Date of publication of application: 18.12.2024
(73) Proprietor: BIOTRONIK SE & Co. KG, 12359 Berlin (DE)
(72) Inventor: WEISS, Ingo, 12435 Berlin (DE); DÖRR, Thomas, 12437 Berlin (DE); PIESKE, Thomas, 12351 Berlin (DE)
(74) Representative: Biotronik Corporate Services SE
(86) International application number: PCT/EP2023/052991
(87) International publication number: WO 2023/152131

(56) References cited:
- EP-A1- 2 465 575
- US-A- 4 399 819
- US-A1- 2004 176 815
- US-A1- 2017 246 459
- US-A1- 2021 128 919
- US-B2- 10 610 693

## Description

The present invention relates to an active implantable medical device according to the preamble of claim 1 and to a method for manufacturing such an active implantable medical device according to the preamble of claim 15.

The term "active implantable medical device" is well known to a person skilled in the art. An "active medical device" is typically defined to be any medical device relying for its functioning on a source of electrical energy or any source of power other than that directly generated by the human body or gravity.

An "active implantable medical device" is typically defined to be any active medical device which is intended to be totally or partially introduced, surgically or medically, into the human body or by medical intervention into a natural orifice, and which is intended to remain after the procedure.

Further details on active implantable medical devices can be found, e.g., in the consolidated text of the Council Directive 90/385/EEC of 20 June 1990 on the approximation of the laws of the Member States relating to active implantable medical devices with subsequent amendments in the version published on 11 October 2007. The consolidated text of this Council Directive is freely accessible under the following link: https://eur-lex.europa.eu/legal-content/EN/TXT/?uri=CELEX:01990L0385-20071011

For mounting the components received within the housing of an active implantable medical device, mounting frames are used according to prior art solutions. These mounting frames require a lot of space between the individual components to be connected and also between the components to be connected and an inner wall of the housing. Such mounting frames are an implant-specific component that requires additional material, costs and logistic efforts.

In document US 2021/128919 A1 a system and method for embedded electronics within a medical implant is disclosed. The system comprises: an implant body; a circuitry surface, containing at least one electronic component, wherein the circuitry surface is at least partially embedded within a defined cavity of the implant along at least one path; sheathing, comprising a protective structure, wherein the at least partially embedded portion of the circuitry surface is enclosed within the sheathing; electronic components connected to, or directly on, the circuitry surface, comprising a set of electrodes and an antenna; and wiring, connecting the circuitry surface and electronic components. The system and method may further include a casing, wherein the casing is a sealed structure directly connected to the implant body including a printed a circuit board (PCB) contained within the casing itself.

In document US 10 610 693 B2 an encapsulation configuration for electronic components in a flexible implantable medical device is disclosed. The encapsulation configuration includes at least one set of folded circuit boards and a filler material, the set of folded circuit boards including a plurality of circuit boards and a plurality of connection cables, each one of the circuit boards including at least one electronics component, each one of the circuit boards having a generally rectangular shape, the connection cables electrically coupling adjacent ones of the circuit boards and the circuit boards being folded over one another in a pleated manner, the filler material surrounding the set of folded circuit boards, the filler material and the set of folded circuit boards together having a cylindrical shape, the set of folded circuit boards being positioned lengthwise in the cylindrical shape and the electronics component being positioned on the set of folded circuit boards to achieve optimal volume consumption in the electronics encapsulation.

It is an object of the present invention to provide an active implantable medical device in which individual components are mechanically stable connected to each other in a spacesaving manner, wherein the connection can be easier realized than according to the mounting frame solutions known from prior art.

This object is achieved with an active implantable medical device having the features of claim 1. Such an active implantable medical device comprises a housing as well as a first component and a second component. The first component and the second component are received within the housing. They are stabilized and mechanically linked to each other by a connecting element to form a composite unit. According to an aspect of the presently claimed invention, the connecting element has a film-like (foil-like) shape with a thickness lying in a range of from 0.01 mm to 0.1 mm, in particular from 0.02 mm to 0.09 mm, in particular from 0.03 mm to 0.08 mm, in particular from 0.04 mm to 0.07 mm, in particular from 0.05 mm to 0.06 mm. The connecting element adheres at least section-wise to the first component and to the second component.

Due to this adhesive connection between the connecting element on the one hand and the first component and the second component on the other hand, a mechanically stable and stabilized connection between the two components is established. At the same time, no mounting frame is necessary. Due to the small thickness of the connecting element, much less space than in case of using a mounting frame is required to form a mechanic stabilization and connection between the first component and the second component. The film-like (or foil-like) shape of the connecting element is a particularly cheap possibility for establishing such a mechanically stable connection between the first component and the second component. Furthermore, it does not require any implant-specific shape like a classic mounting frame known from prior art does. Consequently, the logistic effort is significantly reduced since the connecting element can be universally applied to different kinds of active implantable medical devices and for connecting many different kinds of first and second components housed within the housing of such active implantable medical device.

Since the connection between the first component and the second component is realized by the connecting element and not by a classic mounting frame, the connection between the first component and the second component can also be denoted as mounting frame-free connection. In an embodiment, the active implantable medical device is a mounting frame-free active implantable medical device, i.e., it does not comprise a mounting frame for mounting different components inside its housing.

In an embodiment, the active implantable medical device is an implantable pulse generator (IPG), an implantable cardioverter-defibrillator (ICD), a device for cardiac resynchronization therapy (CRT), or an implantable cardiac monitor. An appropriate cardiac monitor is a loop recorder.

In an embodiment, the connecting element has at least in the area, in which an adhesive connection is established between the connecting element and the first component or the second component, respectively, a similar surface shape like the respective component with which it is connected. Such an adaptation of the shape of the connecting element to the outer shape of the respective component facilitates a strong adhesion that can be easily established between the connecting element and the first component and/or the second component.

As outlined above, the thickness of the connecting element lies in a range of from 0.01 mm to 0.1 mm and is thus significantly smaller than the other dimensions of the connecting element, i.e. the length of the connecting element and the width of the connecting element. The term "significantly smaller" denotes a difference of at least one order of magnitude, in particular at least two orders of magnitude, in particular at least three orders of magnitude.

In an embodiment, the connecting element increases the bending moment of the composite unit. This results in a mechanic stabilization of the first component and the second component in the composite unit with respect to the stability or flexibility of the first component or the second component when not being mechanically linked to each other.

In an embodiment, the connecting element is arranged and designed to exert a tensioning force onto the first component and the second component when a deforming force is exerted onto the composite unit. Due to this tensioning force, the mechanic connection and/or the stiffening of the structure of the composite unit is increased. In this context, it is possible that the connecting element is, in a non-stressed state, practically force-free. The tensioning force only occurs upon trying to deform the composite unit.

In an embodiment, the connecting element is applied to the first component and to the second component under a (light) pretensioning to already exert a tensioning force onto the first component and the second component if no external force is applied onto the composite unit. This additionally increases the stiffness and stability of the composite unit.

In an embodiment, the connecting element covers at least 20 %, in particular at least 30 %, in particular at least 40 %, in particular at least 50 % of the surface of the first component and/or the second component. Thus, the connecting element is applied onto the first component and the second component in a large-scale manner. This serves for a particularly appropriate connection and stabilization of the first component and the second component. Particularly appropriate surface ranges of the first component and/or the second component that are covered by the connecting element are areas lying in a range of from 20 % to 100 %, in particular from 30 % to 90 %, in particular from 40 % to 80 %, in particular from 50 % to 60 %, in particular from 70 % to 75 %.

**In** an embodiment, the connecting element comprises at least one aperture. Such an aperture can also be denoted as opening or through hole in the connecting element and extends from a first side to a second side of the connecting element, wherein the first side and the second side are separated from each other by the thickness of the connecting element. By providing a plurality of apertures, it is possible to design the connecting element with a big circumference, but a rather small overall surface. This reduces the amount of material being necessary for manufacturing the connecting element, but can result - depending on the number and arrangement of the apertures - in basically unaltered connecting properties of the connecting element. Expressed in other words, such an aperture does not necessarily reduce the suitability of the connecting element to connect the first component and the second component with each other in a mechanically stable manner.

**In** an embodiment, the active implantable medical device comprises a plurality (i.e., two or more) of connecting elements.

**In** an embodiment, the connecting elements are arranged distinct to each other, e.g., in parallel to each other. **In** an embodiment, the active implantable medical device comprises at least two connecting elements that are arranged in parallel to each other at the maximum possible distance on or around the first component and the second component. By such an arrangement, a particularly high stiffness of the composite unit is achieved.

In an embodiment, the connecting elements of the plurality of connecting elements are arranged in an at least partially crossing pattern. I.e., in this embodiment, at least some of the connecting elements are diagonally arranged to each other so that at least two of the connecting elements cross each other in a crossing area.

In an embodiment, the connecting elements are arranged in form of a grid. Such a grid is a specific example of an at least partially crossing pattern. A grid serves for a particular appropriate force introduction onto and force transmission between the first component and the second component. Consequently, a particularly appropriate stabilization of the first component and the second component can be achieved by accordingly arranged connecting elements.

In an embodiment, the one or more connecting element is arranged in form of a closed loop. For this purpose, the connecting element may be wound around the first component and the second component in an appropriate pattern.

In an embodiment, the one or more connecting element is configured to be tensioned and arrested like a tension belt, in particular like a ratchet tie-down strap.

In an embodiment, the one or more connecting element is configured to be arrested like a cable connector (i.e., with the help of a latching mechanism).

In an embodiment, the connecting element is particularly stiff to be able to stabilize the composite unit of the first component and the second component in a particularly appropriate way. In this embodiment, the composite unit bends under dead load by not more than 20°, in particular not more than 15°, in particular not more than 10°, in particular not more than 5°. Appropriate deformation angles due to a deformation under dead load lie in a range of from 0° to 20°, in particular from 5° to 15°, in particular from 10° to 12°. Appropriate examples for particularly stiff connecting elements are embodiments in which the connecting element is realized in form of a metal sheet, in form of a stiff plastic foil, or in form of a laminate such as a fiber-reinforced plastic material or a carbon fiber reinforced plastic material.

The deformation angle can be determined in different ways, leading all to the same result.

According to a first variant, the deformation angle is measured by measuring an angle between a first straight line extending along a first lateral surface of the first component and a second straight line extending along a second lateral surface of the second component, wherein the first lateral surface and the second lateral surface face each other.

According to a second variant, the deformation angle is measured between a first straight line extending along a first top surface of the first component and a second straight line extending along a second top surface of the second component, wherein the first top surface and the second top surface are - without any influence of the dead load of the first component and/or the second component - preferably flush to each other.

According to a third variant, the deformation angle is measured between a first surface normal of the first component and a second surface normal of the second component. Without any influence of the dead load of the first component and/or the second component, the first surface normal and the second surface normal are parallel to each other.

According to a fourth variant, the deformation angle is measured by determining a supplementary angle between a first section of the connecting element and a second section of the connecting element, wherein the first section extends parallel to a first top surface of the first component and the second section extends parallel to a second top surface of the second component, wherein the first top surface and the second top surface are - without any influence of the dead load of the first component and/or the second component - preferably flush to each other.

According to a fifth variant, the deformation angle between the first component and the second component can be determined with the help of an angled stop in analogy to DIN 875-1:2005 (which refers to measurements of a 90° angle with an angled stop). The procedure described in DIN 875-1:2005 can be easily transferred from measuring a 90° angle to the maximum allowed deformation angle according to embodiments of the present invention.

In an embodiment, the connecting element is particularly tensile-proof. For this purpose, it allows an elastic deformation of not more than 5 % per 1 N, in particular not more than 1 % per 1 N, in particular not more than 0.1 % per 1 N. Appropriate values for the elastic deformation lie in a range of from 0.01 % per 1 N to 5 % per 1 N, in particular from 0.05 % per 1 N to 4 % per 1 N, in particular from 0.1 % per 1 N to 3 % per 1 N, in particular from 0.5 % per 1 N to 2 % per 1 N, in particular from 0.75 % per 1 N to 1 % per 1 N.

The elastic deformation can be determined by Hooke's law from the determined elongation of the connecting element and the applied force. Appropriate methods for measuring the elastic deformation can be performed, e.g., in analogy to the methods described in DIN EN ISO 527-1:2012.

In an embodiment, the connecting element adheres to the first component and to the second component by gluing, by a hook-and-loop fastening, by a vacuum cup connection, by friction, by brazing, by welding, by a magnetic connection (established via magnets or magnetic properties of the connection element and the first component and/or the second component), and/or a snap action connection such as realized by a push button, a hook, and/or a buckle.

In an embodiment, the connecting element adheres to the first component and to the second component by gluing. In this embodiment, the gluing is realized by a self-adhesive gluing layer of the connection element and/or a hot glue and/or an adhesive polymer. In case of a self-adhesive gluing layer of the connection element, the gluing mass is typically based on synthetic rubber, natural rubber and/or an acrylate. An ironing foil is a particularly appropriate example for providing a hot glue. Different kinds of resins are particularly appropriate examples for gluing polymers.

In an embodiment, the glue has electrically insulating properties. Then, it is particularly easy to provide an electric insulation between the connecting element on the one hand and the first component and the second component on the other hand.

In an embodiment, the glue comprises incorporated particles guaranteeing a minimum thickness of the glue and therewith of an insulating distance provided by the glue. Such particles can be, e.g., spherical or fiber-like particles. Glass and other electrical insulation materials are particularly appropriate for forming such particles.

The mechanical stabilization of the composite unit by the connecting element is particularly relevant until the implantation of the active implantable medical device. It is particularly intended to compensate for any load or stress applied to the active implantable medical device when transporting it to the final site of implantation. Consequently, it is not necessary that the connecting element adheres to the first component and the second component over the whole lifetime of the active implantable medical device. Rather, it is generally possible that the connection between the connecting element and the first component and the second component disintegrates over time.

In an embodiment, the active implantable medical device is designed such that the adhesion of the connecting element to the first component and the second component lasts over a longer period of time than minimally required. For this purpose, the glue used for establishing the adhesion between the connecting element and the first component and the second component is stable in a temperature range of from 32 °C to 42 °C, in particular from 33 °C to 41 °C, in particular from 34 °C to 40 °C, in particular from 35 °C to 39 °C, in particular from 36 °C to 38 °C, in particular from 37 °C to 37.5 °C. By such a stability at approximately body temperature, a long-lasting adhesive connection between the connecting element and the first component and the second component can be achieved.

In an embodiment, the glue is at least temporarily stable at temperatures above body temperature, namely at temperatures lying in a range of from 40 °C to 150 °C, in particular from 50 °C to 140 °C, in particular from 60 °C to 130 °C, in particular from 70 °C to 120 °C, in particular from 80 °C to 110 °C, in particular from 90 °C to 100 °C. By such thermal stability, a temporal heating of the active implantable medical device or the first component and/or the second component does not influence the quality of connection between the connecting element and the first component and the second component. Consequently, different treatment steps (such as a temporal heating for disinfection purposes) can be performed on the active implantable medical device without impairing the glue connection between the connecting element and the first component and the second component.

In an embodiment, the connecting element comprises fibers to increase the tensile strength of the connecting element. Glass fibers, carbon fibers and/or aramid fibers are particularly appropriate fibers for this purpose.

In an embodiment, the fibers incorporated into the connecting element are aligned such that they are only effective in the direction of tension, which can be applied for stabilizing the composite unit via a pretensioning, but allow a stretching of the connecting element in other directions. Then, the connecting element can be particularly easy adapted to the shape of the component to which it adheres.

In an embodiment, the connecting element is stretchable at least in that sections in which it is adhered to the first component and/or the second component. Then, the connecting element is able to be finely adapted to the shape of the first component and/or the second component without wrinkling.

In an embodiment, the connecting element does not only provide a mechanic connection to the first component and the second component, but also an electric connection to at least one of the first and second components. Expressed in other words, the connecting element exhibits an electric functionality. For this purpose, it is electrically connected to the first component and/or the second component. The electric functionality can be, e.g., an electric shielding, the functionality of a programming coil or of a film resistor. The establishment of an electric connection is particularly appropriate if the adhesion between the connecting element and the first component and the second component is realized by gluing these elements together.

Particularly in variants in which the mechanical stabilization is realized by a pretensioning, different embodiments are possible to realize these variants in a particular appropriate way. However, these embodiments can also be realized in cases in which no pretensioning is applied by the connecting element.

In a first of these embodiments, the first component and the second component each comprise at least section-wise a surface that can be rolled along. Then, it is particularly easy to apply the film-like connecting element on the first component and on the second component and to apply a pretensioning to the components.

In a second of these embodiments, the first component and the second component touch each other mechanically at one or more contact points. The at least one contact point enables a force transmission between the first component and the second component.

In a third of these embodiments, the first component and the second component touch each other mechanically via at least one supporting element arranged at least partially between the first component and the second component. This supporting element serves for transmitting forces between the first component and the second component.

In a fourth of these embodiments, the supporting element is an electric insulator. Then, any short-circuits otherwise being generally possible between the first component and the second component are effectively avoided.

In a fifth of these embodiments, a support surface is increased by realizing one or more supporting elements as filling structures between the first component and the second component. Optionally, one or more supporting elements can have an insulating and/or damping character. Thus, it is possible to use the one or more supporting elements for damping vibrations and/or noise otherwise occurring due to the direct mechanical contact between the first component and the second component.

According to a sixth of these embodiments, the connection between the first component and the second component is realized such that tensile forces occurring upon exertion of a load onto the first component and/or the second component are effective on both sides of the contact points.

In an embodiment, the connecting element is realized in form of a heat shrink tubing. Such heat shrink tubing can be positioned above the first component and the second component. After applying heat to it, it shrinks and exerts a force onto the first component and the second component, thus mechanically stabilizing both components and forming the composite unit.

In an embodiment, the connecting element does not only adhere to the first component and the second component, but also to the housing of the active implantable medical device. In such a case, not only a mechanically stable composite unit is formed. Rather, this unit is also stabilized or fixed with respect to the housing of the active implantable medical device. This can further increase the longevity of the active implantable medical device since an undesired movement of the first component and the second component within the housing is avoided.

In an embodiment, the connecting element comprises or essentially consists of a mechanically damping material. Then, the connecting element provides an impact protection and/or an elastic suspension of the first component and the second component within the housing of the active implantable medical device. This embodiment is particularly appropriate in case that the first component and/or the second component are sensitive components that should be particularly protected against mechanical impact from the exterior.

In an embodiment, the connecting element comprises or essentially consists of a welding protection element. In this embodiment, welding protection can be provided along the whole connecting element or at least in sections of the connecting element.

In an aspect, the present invention relates to a method for manufacturing an active implantable medical device according to the preceding explanations. This method comprises a step of adhering a connecting element at least section-wise to a first component and a second component of an active implantable medical device to stabilize and mechanically link the first component and the second component to each other. In addition, a composite unit is formed in this way. In this context, the connecting element has a film-like shape with a thickness lying in a range of from 0.01 mm to 0.1 mm.

In an embodiment, a surface of the first component and/or the second component is pretreated prior to applying the connecting element to the first component and to the second component.

In an embodiment, such a pretreatment is performed by applying a primer onto the surface of the first component and/or the second component. Such a primer improves the adhesive properties in particular of a gluing connection between the connecting element on the one hand and the first component and the second component on the other hand.

All embodiments of the active implantable medical device can be combined in any desired way and can be transferred either individually or in any arbitrary combination to the described method. Likewise, all embodiments of the described method can be combined in any desired way and can be transferred either individually or in any arbitrary combination to the described active implantable medical device.

Further details of aspects of the present invention will be explained in the following making reference to exemplary embodiments and accompanying Figures. In the Figures:
- Fig. 1: shows a schematic depiction of a first embodiment of an active implantable medical device;
- Fig. 2: shows a schematic depiction of a second embodiment of an active implantable medical device;
- Fig. 3A: shows a schematic depiction of a third embodiment of an active implantable medical device;
- Fig. 3B: shows a schematic depiction of a fourth embodiment of an active implantable medical device;
- Fig. 4: shows a schematic depiction of a fifth embodiment of an active implantable medical device;
- Fig. 5: shows a schematic depiction of a sixth embodiment of an active implantable medical device; and
- Figures 6A to 6D: show different possibilities for determining a deformation angle between a first component and a second component of an active implantable medical device.

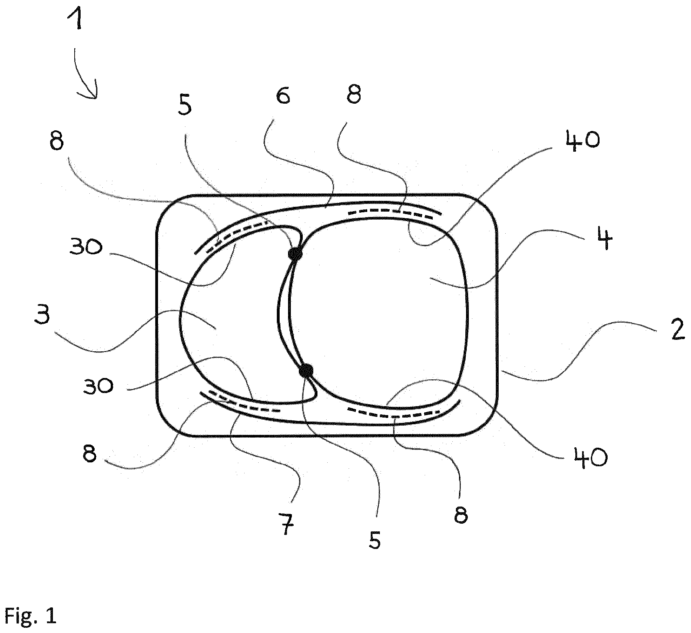

Fig. 1 shows a schematic depiction of a first embodiment of an active implantable medical device 1 comprising a housing 2. Inside the housing 2, a first component 3 and a second component 4 are received. The first component 3 and the second component 4 mechanically contact each other at two contact points 5. A first connecting element 6 and a second connecting element 7 mechanically connect the first component 3 and the second component 4 with each other. For this purpose, the first connecting element 6 and the second connecting element 7 adhere to the first component 3 and to the second component 4 via a bonding agent 8. In the embodiment shown in Fig. 1, the bonding agent 8 is a glue.

The bonding agent 8 is only section-wise applied between the first connecting element 6 and the first component 3 as well as the second component 4 on the one hand and between the second connecting element 7 and the first component 3 as well as the second component 4 on the other hand. Consequently, the first component 3 has two distinct connecting surfaces 30 via which the contact to the first connecting element 6 and the second connecting element 7 is realized. Likewise, the second component 4 has two connecting surfaces 40 that realize a mechanic connection to the first connecting element 6 and the second connecting element 7.

Due to the first connecting element 6 and the second connecting element 7, the first component 3 and the second component 4 form a stabilized composite unit.

If a force is exerted onto the first component 3 and/or the second component 4 acting such that a space between the first component 3 and the second component 4 would be increased and the contact points 5 would cease to exist, the first connecting element 6 and the second connecting element 7 exert an appropriate counter force to keep the first component 3 and the second component 4 tightly together, while maintaining the contact points 5. Thus, the first connecting element 6 and the second connecting element 7 serve for a mechanically stable interconnection between the first component 3 and the second component 4.

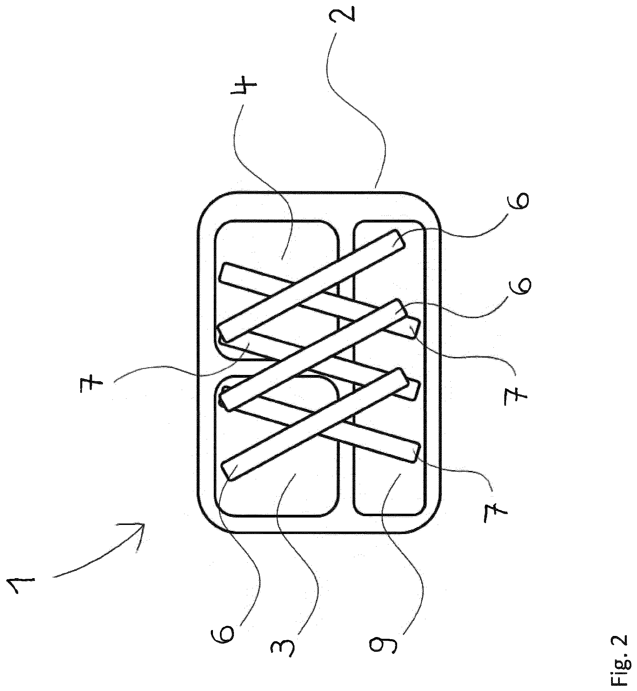

Fig. 2 shows a second embodiment of an active implantable medical device 1. In this and in all following Figures, similar elements will be denoted with the same numeral reference.

The active implantable medical device 1 of Fig. 2 also comprises a housing 2, a first component 3, and a second component 4. Additionally, it comprises a third component 9. The first component 3, the second component 4 and the third component 9 are connected and mechanically stabilized by a plurality of first connecting elements 6 and a plurality of second connecting elements 7. The first connecting elements 6 and the second connecting elements 7 form a grid, i.e., they are arranged in an at least partially crossing pattern. Each of the first connecting elements 6 crosses at least two of the second connecting elements 7. In doing so, a very tight crossing connection pattern is formed that firmly stabilizes the first component 3, the second component 4 and the third component 9 within the housing 1.

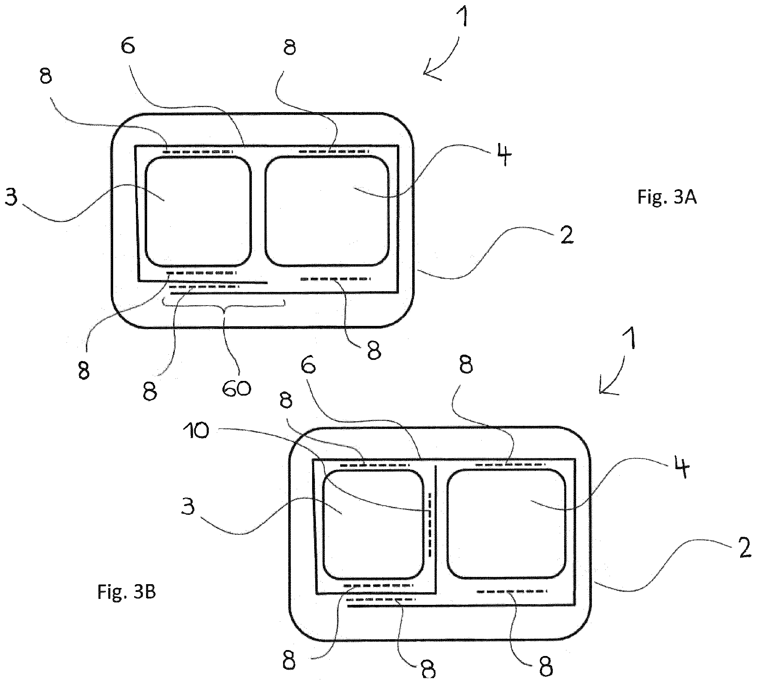

Fig. 3A shows a third embodiment of an active implantable medical device 1. Here, a first component 3 and a second component 4 are received within a housing 2 and are stabilized by a first connecting element 6 that is a single connecting element. To achieve a good stabilization and mechanic connection between the first component 3 and the second component 4, the first connecting element 6 extends around the whole circumference of the composite unit formed by the first component 3 and the second component 4. In addition, in a connecting section 60, a first layer of the first connecting element 6 adheres to a second layer of the first connecting element 6 by the same bonding agent 8 that is also used to adhere the first connecting element 6 to an outer surface of the first component 3 and the second component 4. Due to this connecting section 60, the first connecting element 6 tightly surrounds the first component 3 and the second component 4 and serves for a basically invariable arrangement of the first component 3 and the second component 4 within the housing 1.

Fig. 3B shows a fourth embodiment of an active implantable medical device 1 that is very similar to the third embodiment shown in Fig. 3A. The fourth embodiment also employs a single first connecting element 6 that surrounds the whole circumference of the composite unit formed by the first component 3 and the second component 4. In contrast to the embodiment shown in Fig. 3A, the first connecting element 6 also extends between the first component 3 and the second component 4. In doing so, it prevents a direct physical contact between the first component 3 and the second component 4, while serving for a particularly high mechanic stabilization between the first component 3 and the second component 4.

To achieve a particularly appropriate electric insulation between the first component 3 and the second component 4, an additional insulation layer 10 is placed between the first component 3 and the first connecting element 6 in the region in which the first component 3 would face the second component 4 if the first connecting element 6 was not present. This insulation layer 10 can be made of any appropriate thickness, depending on the specific needs of the respective active implantable medical device 1.

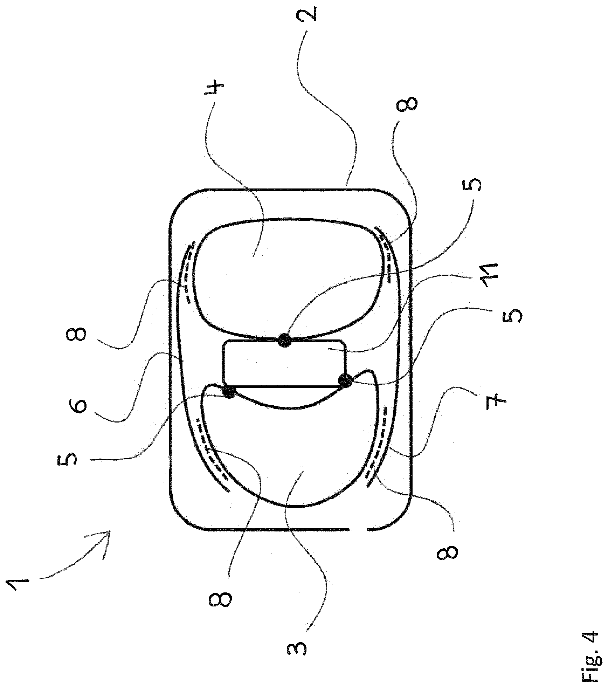

Fig. 4 shows a fifth embodiment of an active implantable medical device 1 that is very similar to the embodiment shown in Fig. 1. Therefore, reference is made to the explanations given above with respect to Fig. 1. In contrast to the embodiment shown in Fig. 1, the embodiment of Fig. 4 comprises a supporting element 11 located between the first component 3 and the second component 4. This supporting element 11 forms two contact points 5 with the first component 3 and a single contact point 5 with the second component 4 (obviously, a different number of contact points could also be established). Due to the contact points 5 between the first component 3 and the supporting element 11 on the one hand or between the second component 4 and the supporting element 11 on the other hand, a force can be transmitted from the first component 3 to the second component 4 (and vice versa) via the supporting element 11. The supporting element 11 can have electrically insulating and/or mechanically damping properties according to the concrete needs.

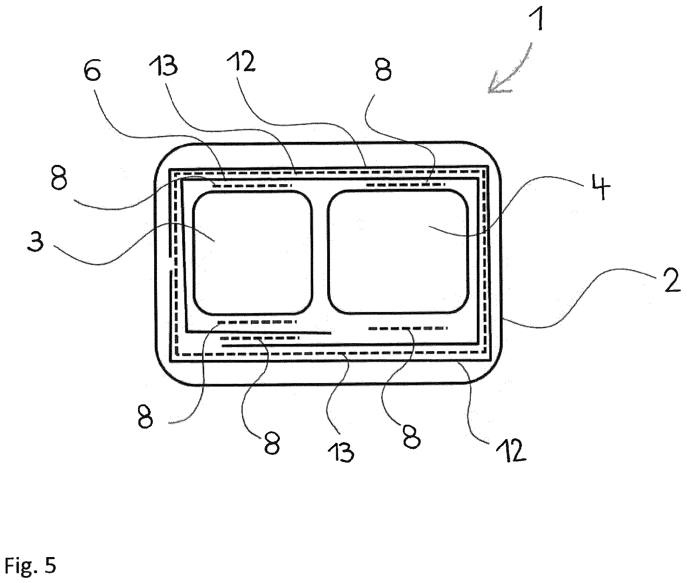

Fig. 5 shows a sixth embodiment of an active implantable medical device that is based on the embodiment shown in Figures 3A and 3B. The first component 3 and the second component 4 are secured to each other with the first connecting element 6 serving as a single connecting element. On an outside of the first connecting element 6 (facing an inside of the housing 1), a welding protection 12 is applied with the help of welding protection bonding agent 13. By this arrangement, a particularly appropriate protection of the first component 3 and the second component 4 against mechanical impacts (e.g., due to transporting the active implantable medical device 1) as well as against thermal impacts (e.g., due to welding processes inside or on an outside of the active implantable medical device 1) is achieved.

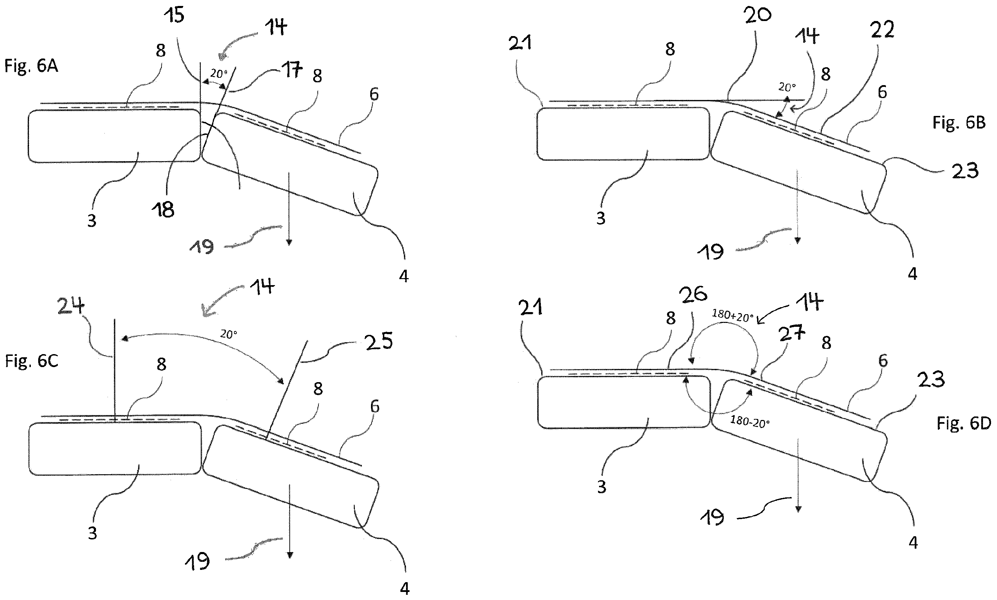

Fig. 6A shows a first variant for determining a deformation angle 14 between a first component 3 and a second component 4 that are connected with a connecting element 6 bonded by a bonding agent 8 to the first component 3 and the second component 4. According to this variant, the deformation angle 14 is measured by measuring an angle between a first straight line 15 extending along a first lateral surface 16 of the first component 3 and a second straight line 17 extending along a second lateral surface 18 of the second component 4, wherein the first lateral surface 16 and the second lateral surface 18 face each other. The deformation angle is formed because of a relative movement between the first component 3 and the second component 4 due to the dead load 19 of the first component 3 and/or the second component 4.

Fig. 6B shows a second variant for determining a deformation angle 14 between a first component 3 and a second component 4 that are connected with a connecting element 6 bonded by a bonding agent 8 to the first component 3 and the second component 4. According to this variant, the deformation angle 14 is measured between a first straight line 20 extending along a first top surface 21 of the first component 3 and a second straight line 22 extending along a second top surface 23 of the second component 4, wherein the first top surface 21 and the second top surface 23 are - without any influence of the dead load 19 of the first component 3 and/or the second component 4 - preferably flush to each other.

Fig. 6C shows a third variant for determining a deformation angle 14 between a first component 3 and a second component 4 that are connected with a connecting element 6 bonded by a bonding agent 8 to the first component 3 and the second component 4. According to this variant, the deformation angle 14 is measured between a first surface normal 24 of the first component 3 and a second surface normal 25 of the second component 4. Without any influence of the dead load 19 of the first component 3 and/or the second component 4, the first surface normal 24 and the second surface normal 25 are parallel to each other.

Fig. 6D shows a fourth variant for determining a deformation angle 14 between a first component 3 and a second component 4 that are connected with a connecting element 6 bonded by a bonding agent 8 to the first component 3 and the second component 4. According to this variant, the deformation angle 14 is measured by determining a supplementary angle between a first section 26 of the connecting element 6 and a second section 27 of the connecting element 6, wherein the first section 26 extends parallel to a first top surface 21 of the first component 3 and the second section 27 extends parallel to a second top surface 23 of the second component 4, wherein the first top surface 21 and the second top surface 23 are - without any influence of the dead load 19 of the first component 3 and/or the second component 4 - preferably flush to each other.

## Claims

1. Active implantable medical device (1) comprising
a housing (2) and
a first component (3) and a second component (4) received within the housing (2), wherein the first component (3) and the second component (4) are stabilized and mechanically linked to each other by a first connecting element (6) and a second connecting element (7) to form a composite unit,
**characterized**
**in that** each of the first connecting element (6) and the second connecting element (7) has a film-like shape with a thickness lying in a range of from 0.01 mm to 0.1 mm and adheres at least section-wise to the first component (3) as well as to the second component (4).

2. Active implantable medical device according to claim 1, **characterized in that** the first connecting element (6) and/or the second connecting element (7) is configured to exert a tensioning force onto the first component (3) and the second component (4) upon exertion of a deforming force onto the composite unit.

3. Active implantable medical device according to claim 1 or 2, **characterized in that** the first connecting element (6) and or the second connecting element (7) comprises at least one aperture.

4. Active implantable medical device according to any of the preceding claims, **characterized in that** the active implantable medical device (1) comprises a plurality of connecting elements (6, 7) that are arranged in an at least partially crossing pattern.

5. Active implantable medical device according to any of the preceding claims, **characterized in that** the composite unit bends under dead load (19) by not more than 20°.

6. Active implantable medical device according to any of the preceding claims, **characterized in that** the first connecting element (6) and or the second connecting element (7) is tensile-proof and allows an elastic deformation of not more than 5 % per 1 N.

7. Active implantable medical device according to any of the preceding claims, **characterized in that** the first connecting element (6) and/or the second connecting element (7) adheres to the first component (3) and to the second component (4) by at least one of gluing, a hook-and-loop fastening, a vacuum cup connection, friction, brazing, welding, a magnetic connection, and a snap-action connection.

8. Active implantable medical device according to claim 7, **characterized in that** the first connecting element (6) and/or the second connecting element (7) adheres to the first component (3) and to the second component (4) by gluing, wherein the gluing is realized by at least one of a self-gluing layer of the connecting element (6, 7), a hot glue, and an adhesive polymer.

9. Active implantable medical device according to any of the preceding claims, **characterized in that** the first connecting element (6) and/or the second connecting element (7) comprises reinforcing fibers.

10. Active implantable medical device according to any of the preceding claims, **characterized in that** the first connecting element (6) and/or the second connecting element (7) exhibits an electric functionality and is electrically connected to at least one of the first component (3) and the second component (4).

11. Active implantable medical device according to any of the preceding claims, **characterized in that** the first component (3) and the second component (4) touch each other at at least one contact point (5).

12. Active implantable medical device according to any of the preceding claims, **characterized in that** the first component (3) and the second component (4) touch each other via at least one supporting element (11) arranged at least partially between the first component (3) and the second component (4).

13. Active implantable medical device according to claim 12, **characterized in that** the supporting element (11) is an electric insulator.

14. Active implantable medical device according to any of the preceding claims, **characterized in that** the first connecting element (6) and/or the second connecting element (7) additionally adheres to the housing (2).

15. Method for manufacturing an active implantable medical device (1) according to any of the preceding claims, **characterized in that** the method comprises the following step:
adhering a first connecting element (6) and a second connecting element (7) at least section-wise to a first component (3) and to a second component (4) of an active implantable medical device (1) to stabilize and mechanically link the first component (3) and the second component (4) to each other and to form a composite unit, wherein each of the first connecting element (6) and the second connecting element ,(7) has a film-like shape with a thickness lying in a range of from 0.01 mm to 0.1 mm.

## Patentansprüche

1. Aktive implantierbare medizinische Vorrichtung (1), umfassend ein Gehäuse (2) und
eine erste Komponente (3) und eine zweite Komponente (4), die innerhalb des Gehäuses (2) aufgenommen sind, wobei die erste Komponente (3) und die zweite Komponente (4) durch ein erstes Verbindungselement (6) und ein zweites Verbindungselement (7) stabilisiert und mechanisch miteinander gekoppelt sind, um eine zusammengesetzte Einheit zu bilden,
**dadurch gekennzeichnet, dass**
jedes von dem ersten Verbindungselement (6) und dem zweiten Verbindungselement (7) eine filmartige Form mit einer Dicke in einem Bereich von 0,01 mm bis 0,1 mm aufweist und zumindest abschnittsweise an der ersten Komponente (3) sowie an der zweiten Komponente (4) haftet.

2. Aktive implantierbare medizinische Vorrichtung nach Anspruch 1, **dadurch gekennzeichnet, dass** das erste Verbindungselement (6) und/oder das zweite Verbindungselement (7) dazu konfiguriert sind, eine Spannkraft auf die erste Komponente (3) und die zweite Komponente (4) bei Ausübung einer Verformungskraft auf die zusammengesetzte Einheit auszuüben.

3. Aktive implantierbare medizinische Vorrichtung nach Anspruch 1 oder 2, **dadurch gekennzeichnet, dass** das erste Verbindungselement (6) und/oder das zweite Verbindungselement (7) mindestens eine Öffnung umfassen.

4. Aktive implantierbare medizinische Vorrichtung nach einem der vorhergehenden Ansprüche, **dadurch gekennzeichnet, dass** die aktive implantierbare medizinische Vorrichtung (1) eine Vielzahl von Verbindungselementen (6, 7) umfasst, die in einem zumindest teilweise kreuzenden Muster angeordnet ist.

5. Aktive implantierbare medizinische Vorrichtung nach einem der vorhergehenden Ansprüche, **dadurch gekennzeichnet, dass** sich die zusammengesetzte Einheit unter einer Eigenlast (19) von nicht mehr als 20° biegt.

6. Aktive implantierbare medizinische Vorrichtung nach einem der vorhergehenden Ansprüche, **dadurch gekennzeichnet, dass** das erste Verbindungselement (6) und/oder das zweite Verbindungselement (7) zugfest sind und eine elastische Verformung von nicht mehr als 5 % pro 1 N ermöglichen.

7. Aktive implantierbare medizinische Vorrichtung nach einem der vorhergehenden Ansprüche, **dadurch gekennzeichnet, dass** das erste Verbindungselement (6) und/oder das zweite Verbindungselement (7) durch mindestens eines von Kleben, einem Klettverschluss, einer Saugnapfverbindung, Reibung, Löten, Schweißen, einer magnetischen Verbindung und einer Schnappverbindung an der ersten Komponente (3) und an der zweiten Komponente (4) haften.

8. Aktive implantierbare medizinische Vorrichtung nach Anspruch 7, **dadurch gekennzeichnet, dass** das erste Verbindungselement (6) und/oder das zweite Verbindungselement (7) durch Kleben an der ersten Komponente (3) und an der zweiten Komponente (4) haften, wobei das Kleben durch mindestens eines von einer selbstklebenden Schicht des Verbindungselements (6, 7), einem Heißkleber und einem Klebstoffpolymer umgesetzt wird.

9. Aktive implantierbare medizinische Vorrichtung nach einem der vorhergehenden Ansprüche, **dadurch gekennzeichnet, dass** das erste Verbindungselement (6) und/oder das zweite Verbindungselement (7) Verstärkungsfasern umfassen.

10. Aktive implantierbare medizinische Vorrichtung nach einem der vorhergehenden Ansprüche, **dadurch gekennzeichnet, dass** das erste Verbindungselement (6) und/oder das zweite Verbindungselement (7) eine elektrische Funktion aufweisen und elektrisch mit mindestens einer von der ersten Komponente (3) und der zweiten Komponente (4) verbunden sind.

11. Aktive implantierbare medizinische Vorrichtung nach einem der vorhergehenden Ansprüche, **dadurch gekennzeichnet, dass** die erste Komponente (3) und die zweite Komponente (4) an mindestens einem Kontaktpunkt (5) einander berühren.

12. Aktive implantierbare medizinische Vorrichtung nach einem der vorhergehenden Ansprüche, **dadurch gekennzeichnet, dass** die erste Komponente (3) und die zweite Komponente (4) über mindestens ein Stützelement (11), das zumindest teilweise zwischen der ersten Komponente (3) und der zweiten Komponente (4) angeordnet ist, einander berühren.

13. Aktive implantierbare medizinische Vorrichtung nach Anspruch 12, **dadurch gekennzeichnet, dass** das Stützelement (11) ein elektrischer Isolator ist.

14. Aktive implantierbare medizinische Vorrichtung nach einem der vorhergehenden Ansprüche, **dadurch gekennzeichnet, dass** das erste Verbindungselement (6) und/oder das zweite Verbindungselement (7) zusätzlich an dem Gehäuse (2) haften.

15. Verfahren zum Herstellen einer aktiven implantierbaren medizinischen Vorrichtung (1) nach einem der vorhergehenden Ansprüche, **dadurch gekennzeichnet, dass** das Verfahren den folgenden Schritt umfasst:
Haften eines ersten Verbindungselements (6) und eines zweiten Verbindungselements (7) zumindest abschnittsweise an eine erste Komponente (3) und an eine zweite Komponente (4) einer aktiven implantierbaren medizinischen Vorrichtung (1), um die erste Komponente (3) und die zweite Komponente (4) zu stabilisieren und mechanisch aneinander zu koppeln und eine zusammengesetzte Einheit zu bilden, wobei jedes von dem ersten Verbindungselement (6) und dem zweiten Verbindungselement (7) eine filmartige Form mit einer Dicke in einem Bereich von 0,01 mm bis 0,1 mm aufweist.

## Revendications

1. Dispositif médical implantable actif (1) comprenant
un boîtier (2) et
un premier composant (3) et un second composant (4) reçus au sein du boîtier (2), dans lequel le premier composant (3) et le second composant (4) sont stabilisés et liés mécaniquement l'un à l'autre par un premier élément de liaison (6) et un deuxième élément de liaison (7) pour former une unité composite,
**caractérisé**
**en ce que** chacun parmi le premier élément de liaison (6) et le deuxième élément de liaison (7) est en forme de film avec une épaisseur située dans une plage de 0,01 mm à 0,1 mm et adhère au moins par sections au premier composant (3) ainsi qu'au second composant (4).

2. Dispositif médical implantable actif selon la revendication 1, **caractérisé en ce que** le premier élément de liaison (6) et/ou le deuxième élément de liaison (7) est configuré pour exercer une force de tension sur le premier composant (3) et le second composant (4) lors de l'exercice d'une force de déformation sur l'unité composite.

3. Dispositif médical implantable actif selon la revendication 1 ou 2, **caractérisé en ce que** le premier élément de liaison (6) et/ou le deuxième élément de liaison (7) comprend au moins une ouverture.

4. Dispositif médical implantable actif selon l'une quelconque des revendications précédentes, **caractérisé en ce que** le dispositif médical implantable actif (1) comprend une pluralité d'éléments de liaison (6, 7) qui sont agencés en un motif au moins partiellement croisé.

5. Dispositif médical implantable actif selon l'une quelconque des revendications précédentes, **caractérisé en ce que** l'unité composite se courbe sous une charge statique (19) de pas plus de 20°.

6. Dispositif médical implantable actif selon l'une quelconque des revendications précédentes, **caractérisé en ce que** le premier élément de liaison (6) et/ou le deuxième élément de liaison (7) est résistant à la tension et permet une déformation élastique ne dépassant pas 5 % par 1 N.

7. Dispositif médical implantable actif selon l'une quelconque des revendications précédentes, **caractérisé en ce que** le premier élément de liaison (6) et/ou le deuxième élément de liaison (7) adhère au premier composant (3) et au second composant (4) par au moins un parmi un collage, une fixation velcro, une liaison par ventouses, des frottements, un brasage, un soudage, une liaison magnétique, et une liaison par encliquetage.

8. Dispositif médical implantable actif selon la revendication 7, **caractérisé en ce que** le premier élément de liaison (6) et/ou le deuxième élément de liaison (7) adhère au premier composant (3) et au second composant (4) par collage, dans lequel le collage est réalisé par au moins un parmi une couche autocollante de l'élément de liaison (6, 7), une colle à chaud et un polymère adhésif.

9. Dispositif médical implantable actif selon l'une quelconque des revendications précédentes, **caractérisé en ce que** le premier élément de liaison (6) et/ou le deuxième élément de liaison (7) comprend des fibres de renfort.

10. Dispositif médical implantable actif selon l'une quelconque des revendications précédentes, **caractérisé en ce que** le premier élément de liaison (6) et/ou le deuxième élément de liaison (7) présente une fonctionnalité électrique et est connecté électriquement à au moins un parmi le premier composant (3) et le second composant (4).

11. Dispositif médical implantable actif selon l'une quelconque des revendications précédentes, **caractérisé en ce que** le premier composant (3) et le second composant (4) se touchent en au moins un point de contact (5).

12. Dispositif médical implantable actif selon l'une quelconque des revendications précédentes, **caractérisé en ce que** le premier composant (3) et le second composant (4) se touchent par l'intermédiaire d'au moins un élément de soutien (11) agencé au moins partiellement entre le premier composant (3) et le second composant (4).

13. Dispositif médical implantable actif selon la revendication 12, **caractérisé en ce que** l'élément de soutien (11) est un isolant électrique.

14. Dispositif médical implantable actif selon l'une quelconque des revendications précédentes, **caractérisé en ce que** le premier élément de liaison (6) et/ou le deuxième élément de liaison (7) adhère de plus au boîtier (2).

15. Procédé de fabrication d'un dispositif médical implantable actif (1) selon l'une quelconque des revendications précédentes, **caractérisé en ce que** le procédé comprend les étapes suivantes :
faire adhérer un premier élément de liaison (6) et un deuxième élément de liaison (7) au moins par sections à un premier composant (3) et à un second composant (4) d'un dispositif médical implantable actif (1) pour stabiliser et lier mécaniquement le premier composant (3) et le second composant (4) l'un à l'autre et pour former une unité composite, dans lequel chacun parmi le premier élément de liaison (6) et le deuxième élément de liaison (7) est en forme de film avec une épaisseur située dans une plage de 0,01 mm à 0,1 mm.
